# EUROPEAN PATENT APPLICATION

(11) **EP 3 066 983 A1**
(43) Date of publication of application: **14.09.2016**
(21) Application number: 14859949.1
(22) Date of filing: 06.11.2014
(51) Int. Cl.: A61B 6/00, A61B 6/08

(54) **X-RAY IMAGING DEVICE INCLUDING PLURALITY OF X-RAY SOURCES**

(30) Priority: 06.11.2013 KR 20130134235; 29.08.2014 KR 20140114114
(71) Applicant: Rayence Co., Ltd., Hwaseong-si, Gyeonggi-do 445-170 (KR)
(72) Inventor: BEAK, In-Jae, Hwaseong-si Gyeonggi-do 445-170 (KR); KIM, Tae-Woo, Hwaseong-si Gyeonggi-do 445-170 (KR); LEE, Han-Sung, Hwaseong-si Gyeonggi-do 445-170 (KR)
(74) Representative: Markfort, Iris-Anne Lucie
(86) International application number: PCT/KR2014/010618
(87) International publication number: WO 2015/069039

(57) **Abstract**

The present invention relates to an X-ray imaging device including a plurality of X-ray sources. The X-ray image photographing device, according to the present invention, is an X-ray imaging device comprising an X-ray generation unit and a detector having an object being inspected placed therebetween and are arranged to face each other, wherein the X-ray generation unit includes the plurality of X-ray sources which are arranged along at least two rows located at different distances from the object being inspected and respectively irradiate X-rays toward the object being inspected.

## Description

### BACKGROUND

### 1. Technical Field

The present invention relates to an X-ray image obtaining apparatus including a plurality of X-ray sources.

### 2. Related Art

Recently, an X-ray imaging technology is grafted on a semiconductor section, and thus is rapidly developed into a digital X-ray imaging technology having various advantages, such as relatively high resolution, a wide dynamic range, and the easy generation of an electrical signal, and the simple processing and storage of data instead of a conventional analog method using a film. The digital-based imaging technology strongly reflects a clinical and environmental need called an early diagnosis of a disease based on the excellent diagnosability of a digital image.

Accordingly, there has been introduced "digital mammography", that is, a breast-dedicated X-ray photographing technology capable of detecting a breast cancer and a lesion and fine calcification for an early diagnosis by representing the internal structure of the breast, that is, a specimen, in a high-resolution image using the biological tissue contrast ability unique to X-rays. Such digital mammography is rapidly spread due to unique characteristics, such as the magnification of an image, a reduction of a photographing number, an increase of resolution, and the minimization of atomic bombing through control of brightness and contrast ratio, in addition to various advantages of the digital X-ray imaging technology.

Furthermore, in a digital image obtaining apparatus for obtaining a two-dimensional projection image, a diagnosis is not easy if an abnormal portion (lesion) of a specimen is covered by a human tissue, etc. As a solution for such a problem, after images of the specimen are obtained at various angles, a three-dimensional tomosynthesis image of the specimen may be generated by reconfiguring the obtained images. To this end, a computed tomography (CT) or digital breast tomosynthesis (DBT) apparatus, that is, an example of an existing X-ray imaging apparatus, generates multi-directional X-ray projection images by radiating X-rays to a specimen while relatively rotating a single X-ray source around the specimen and generates a three-dimensional image by reconfiguring the multi-directional X-ray projection images.

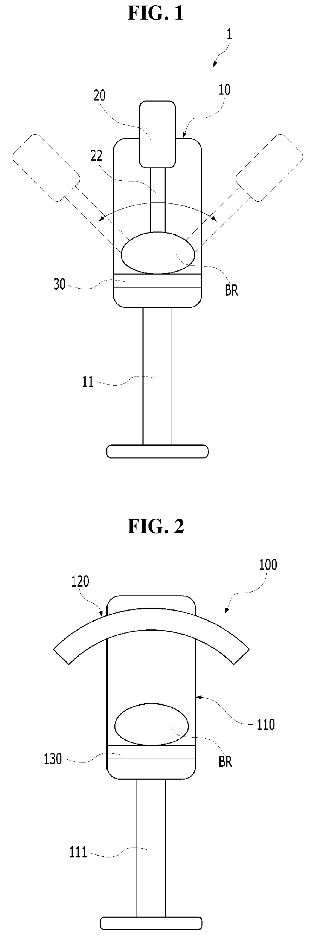

A conventional DBT apparatus is described with reference to FIG. 1. The conventional DBT apparatus 1 a support row 11 of a vertical pillar shape which has a lower end fixed to the bottom, a device body 10 which is installed to move up and down along the support row 11, a detector 30 which is installed on the bottom of the device body 10, and an X-ray generation unit 20 which is rotated around a specimen BR through a rotary support unit 22 rotatably connected to the device body 10.

In such a DBT apparatus 1, when a testee enters a photographing location, the device body 10 moves up and down along the support row 11, and thus the height of the device body 10 is adjusted so that the specimen (e.g., the breast) of the testee is placed on the detector 30. Next, the X-ray generation unit 20 photographs the specimen BR while rotating and moving from the location of the X-ray generation unit 20 indicated by a dotted line on the left side to the location of the X-ray generation unit 20 indicated by a dotted line on the right side by rotating the rotary support unit 22.

In this case, in general, the X-ray photographing is performed in accordance with a continuous-shoot method for photographing the specimen BR when the X-ray generation unit 20 reaches a specific angular interval while rotating around the specimen BR. However, such a continuous-shot method has a problem in that image quality is deteriorated because X-ray photographing is performed while the X-ray generation unit 20 rotates and moves and thus a motion blur phenomenon in which the boundary of an image focused on the detector 30 unclearly appears is generated.

In order to prevent such a motion blur phenomenon, there is used a stop-and-shot method for photographing the specimen BR after the X-ray generation unit 20 is fully stopped at each angular interval in which photographing is to be performed. However, such a stop-and-shot method has problems in that the photographing time is generally lengthened and vibration and noise are generated because X-ray photographing has to be performed in the state in which the X-ray generation unit 20 has been fully stopped at each specific angular interval and then the X-ray generation unit 20 has to move.

### SUMMARY

The present invention has been made to solve the above conventional problems, and an object of the present invention is to provide an X-ray image obtaining apparatus capable of obtaining a high-resolution three-dimensional tomosynthesis image.

An X-ray image obtaining apparatus of the present invention for achieving the above object is an X-ray image obtaining apparatus including an X-ray generation unit and a detector disposed to face each other with a specimen therebetween, wherein the X-ray generation unit includes a plurality of X-ray sources which is disposed in at least two rows at different distances from the specimen and radiates X-rays toward the specimen.

The at least two rows may be arc forms having different radii.

The plurality of X-ray sources disposed in the at least two rows may be crisscross disposed at different angles with respect to the specimen so that the X-rays toward the specimen do not overlap.

The at least two rows may be disposed back and forth and may be arc shapes having the same radius.

The X-ray generation unit may include moving means for adjusting the interval between the at least two rows.

The plurality of X-ray sources may sequentially operate for each row or sequentially operate while changing the rows.

Each of the plurality of X-ray sources may be an electric field emission type X-ray source using a nanostructure material electric field emission type emitter.

### BRIEF DESCRIPTION OF THE DRAWINGS

FIG. 1 is a front view of a conventional DBT apparatus.
FIG. 2 is a front view of an X-ray image obtaining apparatus according to the present invention.
FIG. 3 is a front view of an X-ray generation unit shown in FIG. 2.
FIG. 4(a) is a front view of the X-ray generation unit shown in FIG. 2, and FIG. 4(b) is a plan view of the X-ray generation unit.
FIG. 5 (a) is a front view of the X-ray generation unit shown in FIG. 2, and FIG. 5(b) is a plan view of the X-ray generation unit.
FIG. 6 (a) is a front view of the X-ray generation unit shown in FIG. 2, and FIG. 6(b) is a plan view of the X-ray generation unit.

### DETAILED DESCRIPTION

Hereinafter, preferred embodiments of the present invention are described in detail with reference to the accompanying drawings.

FIG. 2 is a front view of an X-ray imaging apparatus according to the present invention. Referring to FIG. 2, the X-ray image obtaining apparatus 100 according to the present invention includes a support row 111 of a vertical pillar shape and a device body 110 installed in such a way as to move up and down along the support row 111. A generator or X-ray generation unit 120 for radiating X-ray toward a specimen BR is installed on the upper side of the device body 110. A detector 130 which faces the X-ray generation unit 120 and receives the X-rays radiated by the X-ray generation unit 120 is installed on the bottom of the device body 110.

### (First embodiment)

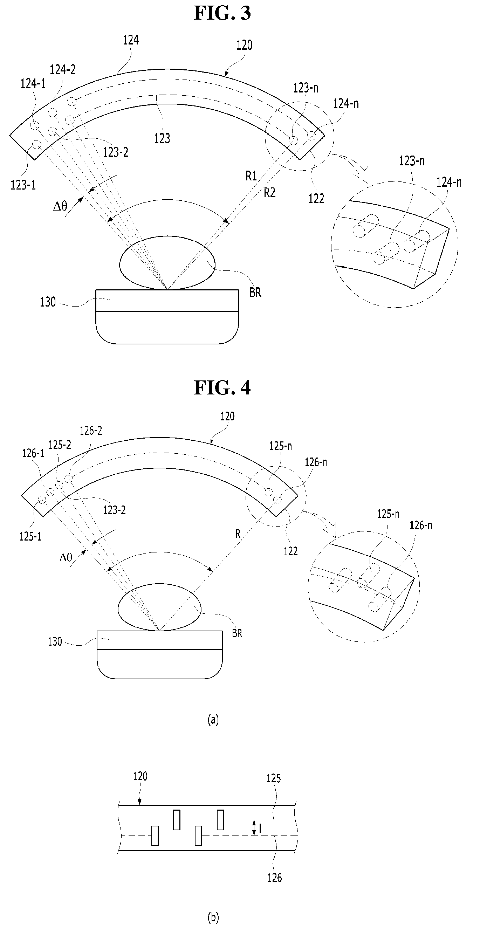

FIG. 3 is a front view of the X-ray generation unit shown in FIG. 2. In accordance with the first embodiment of the present invention shown in FIG. 3, when viewed from the front of the X-ray generation unit 120, that is, in a testee direction, the X-ray generation unit 120 includes a plurality of X-ray sources 123 and 124 which has different distances from the specimen and is arranged to form at least two rows and a mounting unit 122 for detachably installing the plurality of X-ray sources 123 and 124 on the X-ray generation unit 120.

In this case, it is preferred that each of the rows of the X-ray sources 123 and 124 forms an arc shape for the specimen BR, but is not limited thereto. Each of the rows may form a straight-line shape. Furthermore, each of the X-ray sources 123 and 124 may be configured to include X-ray sources of an electric field emission method using a nanostructure material electric field emission emitter, such as a carbon nanotube.

If each of the rows of the X-ray sources 123 and 124 forms an arc shape, as shown in FIG. 3, when viewed from the front of the X-ray generation unit 120, a plurality of X-ray sources 123-1, 123-2, ..., 123-n, 124-1, 124-2, ..., 124-n is crisscross arranged in zigzags with respect to the specimen BR in two rows having different distances from the specimen.

Specifically, when viewed from the front of the X-ray source generation unit 120, assuming that the number of X-ray sources 123 and 124 is 2n, for convenience sake, the 2n X-ray sources 123 and 124 forms two rows each including the n X-ray sources. In this case, the n X-ray sources 123-1, 123-2, ..., 123-n are arranged in a first row along an arc placed at a first distance R1 from the specimen BR. The remaining n X-ray sources 124-1, 124-2, ..., 124-n are arranged in a second row along an arc placed at a second distance R2 from the specimen BR, which is greater than the first distance R1. In this case, it is preferred that the X-ray sources 123 of the first row are spaced apart from each other to the extent that they do not interfere with or hinder X-rays radiated by the X-ray sources 124 of the second row.

Accordingly, the n X-ray sources 123-1, 123-2, ..., 123-n forming the first row and the remaining n X-ray sources 124-1, 124-2, ..., 124-n forming the second row are spaced apart from each other at a specific angular interval Δθ. Accordingly, all the X-ray sources 123 and 124 may radiate X-rays to the specimen BR at different angles.

### (Second embodiment)

FIGS. 4(a) and 4(b) are a front view and plan view of the X-ray generation unit shown in FIG. 2. In accordance with the second embodiment of the present invention shown in FIG. 4, when viewed from the top of the X-ray generation unit 120, the X-ray generation unit 120 includes a plurality of X-ray sources 125 and 126 arranged to form at least two rows and a mounting unit 122 for detachably installing the plurality of X-ray sources 125 and 126 on the X-ray generation unit 120.

As shown in FIG. 4(a), it is preferred that the X-ray sources 125 and 126 form the same arc shape back and forth when viewed from the front of the X-ray generation unit 120, but is not limited thereto. The X-ray sources 125 and 126 may form a straight line form.

As shown in FIG. 4(b), when viewed from the top of the X-ray generation unit 120, X-ray sources 125-1, 125-2, ..., 125-n and 126-1, 126-2, ..., 126-n are crisscross arranged in zigzags back and forth with respect to the specimen BR in at least two rows.

Specifically, assuming that the number of X-ray sources 125 and 126 is 2n, the two n X-ray sources 125 and 126 form two rows each including the n X-ray sources, the n X-ray sources 125-1, 125-2, ..., 125-n arranged in a first row along an arc having a specific radius R from the specimen BR. The remaining n X-ray sources 126-1, 126-2, ..., 126-n are arranged in a second row along an arc having the same radius R from the specimen BR. The first row is spaced apart from the second row at a specific interval I in the direction from the X-ray generation unit 120 to the device body 110, when viewed from the top of the X-ray generation unit 120.

Furthermore, the n X-ray sources 125-1, 125-2, ..., 125-n forming the first row and the remaining n X-ray sources 126-1, 126-2, ..., 126-n forming the second row are spaced apart from each other at a specific angular interval Δθ with respect to the specimen BR. Accordingly, all the X-ray sources 125 and 126 may radiate X-rays to the specimen BR at different angles.

### (Third embodiment)

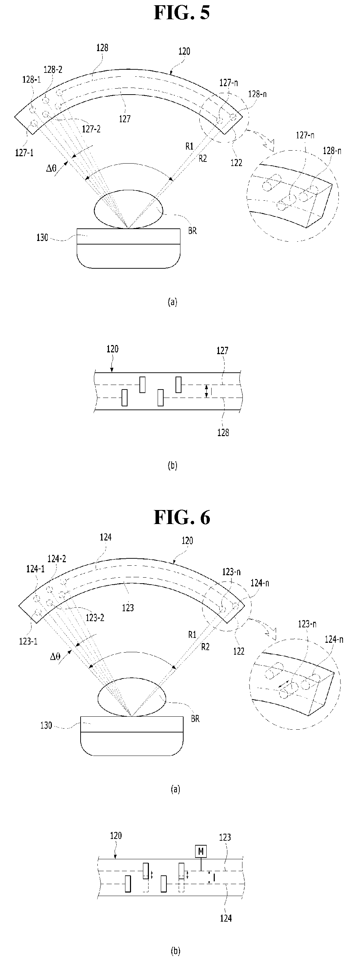

FIGS. 5(a) and 5(b) are a front view and plan view of the X-ray generation unit shown in FIG. 2. In accordance with the third embodiment of the present invention shown in FIG. 5, the X-ray generation unit 120 includes a plurality of X-ray sources 127 and 128 arranged to at least two rows and a mounting unit 122 for detachably installing the X-ray sources 127 and 128 on the X-ray generation unit 120.

When viewed from the front of the X-ray generation unit 120, a plurality of X-ray sources 127-1, 127-2, ..., 127-n and 128-1, 128-2, ..., 128-n is crisscross arranged in zigzags in at least two rows having different distances from the specimen BR, as shown in FIG. 5(a). Even when viewed from the top of the X-ray generation unit 120, the plurality of X-ray sources 127-1, 127-2, ..., 127-n and 128-1, 128-2, ..., 128-n is crisscross arranged in zigzags in the at least two rows back and forth with respect to the specimen BR, as shown in FIG. 5(b).

Specifically, assuming that the number of X-ray sources 127 and 128 is 2n, the two n X-ray sources 127 and 128 form two rows each including the n X-ray sources, when viewed from the front of the X-ray generation unit 120. The n X-ray sources 127-1, 127-2, ..., 127-n are arranged in a first row along an arc placed in a first distance R1 from the specimen BR. The remaining n X-ray sources 128-1, 128-2, ..., 128-n are arranged in a second row along an arc placed at a second distance R2 from the specimen BR, which is greater than the first distance R1. At the same time, even when viewed from the top of the X-ray generation unit 120, the second row is spaced apart from the first row at a specific interval I in the direction from the X-ray generation unit 120 to the device body 110.

In accordance with the array structure of the X-ray sources 127 and 128 according to the third embodiment, X-rays radiated by the X-ray sources 128-1, 128-2, ..., 128-n of the second row reach the specimen BR without interference or hindrance from the X-ray sources 127-1, 127-2, ..., 127-n of the first row because the second row is spaced apart from the first row at the specific interval I when viewed from the top of the X-ray generation unit 120. Accordingly, the plurality of X-ray sources 127 and 128 can be more densely arranged within the X-ray source generation unit 120 because an angular interval Δθ between the X-ray sources 127 and 128 can be reduced to the fullest. As a result, a sharp high-resolution three-dimensional synthesis image can be obtained.

### (Fourth embodiment)

FIGS. 6(a) and 6(b) are a front view and plan view of the X-ray generation unit shown in FIG. 2. The fourth embodiment shown in FIG. 6 is a modified example of the first embodiment, and differences between the fourth embodiment and the first embodiment are chiefly described. The X-ray image obtaining apparatus 100 further includes moving means M for moving the X-ray sources 123-1, 123-2, ..., 123-n of at least one of a first row and a second row so that the X-ray sources 123-1, 123-2, ..., 123-n of the first row are spaced apart from the X-ray sources 124-1, 124-2, ..., 124-n of the second row at a specific interval I, when viewed from the top of the X-ray source generation unit 120.

In this case, it is preferred that the moving means M is a linear motor capable of moving the X-ray sources 123 of at least one of the first row and the second row, but may include a rotary motor.

Various methods may be applied to the X-ray generation unit 120 according to the fourth embodiment depending on purposes. However, for example, in the state in which the first row and the second row have overlapped up and down when viewed from the top of the X-ray source generation unit 120, a first driving method in which the X-ray sources of the first and the second rows sequentially radiate X-rays alternately or the X-ray sources of the first or the second row first sequentially radiate X-rays and the X-ray sources of the other row then sequentially radiate X-rays is possible. In this case, interference or hindrance may be present between the X-ray sources of the first and the second rows depending on the number of X-ray sources, etc. For another example, in the state in which the first row and the second row have overlapped up and down when viewed from the top of the X-ray source generation unit 120, a second driving method in which the X-ray sources 123-1, 123-2, ..., 123-n of the first row first sequentially radiate X-rays toward the specimen BR, the X-ray sources 123-1, 123-2, ..., 123-n of the first row are moved to a location spaced apart from the X-ray sources 124-1, 124-2, ..., 124-n of the second row at a specific interval I, the X-ray sources 124-1, 124-2, ..., 124-n of the second row sequentially radiate X-rays toward the specimen BR, and the X-ray sources 123-1, 123-2, ..., 123-n of the first row are then returned to the original location is possible.

In accordance with the second driving method of the fourth embodiment, as in the third embodiment, the X-ray sources 123 belonging to the first row are spaced apart from the second row at the specific interval I when viewed from the top of the X-ray generation unit 120 and are moved. Accordingly, X-rays radiated by the X-ray sources 124-1, 124-2, ..., 124-n forming the second row reach the specimen BR without interference or hindrance from the X-ray sources 127-1, 127-2, ..., 127-n forming the first row. Accordingly, the X-ray sources 123 and 124 can be arranged more densely within the X-ray source generation unit 120 because an angular interval Δθ between the X-ray sources 123 and 124 can be reduced to the fullest. Furthermore, unlike in the third embodiment, in the fourth embodiment, when three-dimensional stereoscopic images are synthesized, it is not necessary to correct a difference between images attributable to the interval I between the first row and the second row. Accordingly, a sharper high-resolution three-dimensional synthesis image can be obtained.

In the preferred embodiments described so far, the plurality of X-ray sources has been illustrated as being arranged in 2 rows, but is not limited thereto. The plurality of X-ray sources may be arranged in 3 rows or 4 rows or more rows.

Although a specific X-ray source malfunctions, a high- resolution three-dimensional synthesis image can be obtained without a problem by performing photographing using only the X-ray sources of other rows other than a corresponding row or by replacing the fail specific X-ray source with an adjacent another X-ray source of an adjacent another row, performing photographing, and then correcting an image.

The X-ray generation unit 120 may be detachably installed on the device body 110, the mounting unit 122 may be detachably installed on the X-ray generation unit 120, and the X-ray sources may be detachably installed on the mounting unit 122. Accordingly, a user can selectively install the mounting unit, having various patterns (e.g., the interval between the X-ray sources and the distribution, array, etc. of the X-ray sources), on the X-ray generation unit 120 according to a photographing purpose in response to required resolution and/or a different type of specimen, and can also easily replace and repair an X-ray source when it fails.

Tomosynthesis photographing using the X-ray image obtaining apparatus 100 according to the first to the fourth embodiments of the present invention is described below.

First, when a testee in a stand-up or standing state is in-situ placed at the photographing location of the X-ray image obtaining apparatus 100, the device body 110 moves up and down along the row 111, and thus the device body 110 is positioned so that the specimen BR is placed on the detector 130. In this case, in order to prevent an abnormal portion within the specimen BR from being covered by a mammary gland tissue, etc., it is preferred that the specimen BR placed on the detector 130 is pressurized by a compression pad (not shown).

Next, the plurality of X-ray sources sequentially operates for each row or sequentially operates while changing their rows and radiates X-rays to the specimen BR at different angles. The radiated X-rays pass through the specimen BR and are received by the detector 130. The detector 130 generates an electrical signal for each location, which is proportional to the amount of the received incident X-rays, reads the electrical signals and location information, and obtains X-ray images of the specimen BR obtained at the respective angles by processing the read electrical signals and location information using an image processing algorithm.

Thereafter, a high-resolution three-dimensional synthesis image can be obtained by synthesizing the X-ray images of the specimen BR obtained at the different angles using a tomosynthesis method well known to those skilled in the art to which the present invention pertains.

The X-ray image obtaining apparatus according to the present invention can obtain a high-resolution three-dimensional tomosynthesis image and thus perform an accurate lesion diagnosis because X-ray photographing is rapidly performed on a specimen at various angles through the plurality of X-ray sources arranged according to a specific rule.

Furthermore, reliability of a product is improved because a high-resolution three-dimensional tomosynthesis image can be obtained although some of the plurality of X-ray sources malfunction.

Furthermore, the X-ray image obtaining apparatus according to the present invention does not generate any vibration and noise because it does not need to have additional movable parts for rotation driving.

An example in which the X-ray image obtaining apparatus according to the present invention has been used as a mammography apparatus, that is, an X-ray apparatus for photographing the breast, has been described above, but the scope of the present invention is not limited to a device used for such a purpose. That is, the X-ray image obtaining apparatus of the present invention can be applied to all types of X-ray photographing apparatuses for obtaining projection images of a specimen using X-rays. Those skilled in the art to which the present invention pertains may easily understand that the technical scope of the present invention covers an X-ray photographing apparatus of such a type. Furthermore, the X-ray image obtaining apparatus of the present invention should be construed as covering all modifications or variations derived from the meaning and scope of the appended claims and their equivalents.

## Claims

1. An X-ray image obtaining apparatus including an X-ray generation unit and a detector disposed to face a specimen, wherein:
the X-ray generation unit comprises a plurality of X-ray sources in at least two rows at different distances from the specimen and radiates X-rays toward the specimen.

2. The X-ray image obtaining apparatus of claim 1, wherein the at least two rows are arc forms having different radii.

3. The X-ray image obtaining apparatus of claim 1, wherein the plurality of X-ray sources in the at least two rows is crisscross disposed at different angles with respect to the specimen so that the X-rays toward the specimen do not overlap.

4. The X-ray image obtaining apparatus of claim 1, wherein the at least two rows are disposed back and forth and are arc shapes having an identical radius.

5. The X-ray image obtaining apparatus of any one of claims 1 to 3, wherein the X-ray generation unit comprises moving means for adjusting an interval between the at least two rows.

6. The X-ray image obtaining apparatus of claim 1, wherein the plurality of X-ray sources sequentially operates for each row or sequentially operates while changing the rows.

7. The X-ray image obtaining apparatus of claim 1, wherein each of the plurality of X-ray sources is an electric field emission type X-ray source using a nanostructure material electric field emission type emitter.
